Europäisches Patentamt

**European Patent Office**

**Office européen des brevets**

(19)

(11) Numéro de publication: **0 147 265**

**A1**

(12)

# DEMANDE DE BREVET EUROPEEN

(21) Numéro de dépôt: **84402275.6**

(22) Date de dépôt: **12.11.84**

(51) Int. Cl.⁴: **A 61 B 17/56**

(30) Priorité: **14.11.83 FR 8318057**

(43) Date de publication de la demande:
**03.07.85 Bulletin 85/27**

(84) Etats contractants désignés:
**AT BE CH DE GB IT LI LU NL SE**

(71) Demandeur: **MEDICALEX Société Anonyme dite:**
**39, Rue Croulebarbe**
**F-75013 Paris(FR)**

(72) Inventeur: **Levy, Aldo**
**10, Rue Vandrezanne**
**F-75013 Paris(FR)**

(74) Mandataire: **Gillard, Marie-Louise et al,**
**Cabinet Beau de Loménie 55, Rue d'Amsterdam**
**F-75008 Paris(FR)**

(54) **Matériel d'ostéosynthèse pour le traitement des fractures du fémur.**

(57) L'invention concerne un matériel d'ostéosynthèse pour le traitement des fractures du fémur.

La plaque d'ostéosynthèse selon l'invention comprenant une plaque (1) et un élément de fixation (2), ladite plaque présentant des trous (8) et (9) et un trou taraudé (5), présente par rapport à l'axe de l'élément de fixation (2) deux inclinaisons α et β, l'inclinaison β de l'extrémité de la plaque possédant le trou taraudé (5) étant inférieure à l'inclinaison α de l'autre extrémité de la plaque, ladite inclinaison α correspondant sensiblement à l'angle α de correction.

Fig-1

EP 0 147 265 A1

## Matériel d'ostéosynthèse pour le traitement des fractures du fémur

La présente invention concerne des perfectionnements apportés au matériel d'ostéosynthèse destiné notamment au traitement des fractures du fémur. Elle a plus particulièrement pour objet une nouvelle plaque d'ostéosynthèse et les moyens pour la mise en place ou l'extraction d'une telle plaque.

Un exemple de plaques couramment utilisées pour monter solidement des ostéosynthèses à plan de coupe oblique, par exemple pour réduire les fractures du col du fémur, est décrit dans le brevet FR 1 443 965.

Ces plaques, généralement du type clou-plaque ou du type plaque-à-vis, sont munies d'un trou taraudé et présentent, par rapport à l'élément de fixation dont elles sont solidaires, une seule inclinaison $\alpha$, qui correspond sensiblement à l'angle $\alpha$ entre le col du fémur et la diaphyse fémorale. Par "élément de fixation", on désigne dans la présente description, le corps d'un clou-plaque, l'oeillet-plaque d'une plaque à vis ou tout autre élément utilisé pour fixer une plaque dans une ostéosynthèse à plan de coupe oblique. Ces plaques n'épousent pas correctement la structure de l'os présentant une fracture à réduire.

Contrairement aux plaques de l'art antérieur, les plaques selon l'invention, qui peuvent être aussi bien des plaques à vis que des clou-plaques présentent essentiellement deux inclinaisons par rapport à l'axe de l'élément de fixation, tel que défini précédemment, l'inclinaison $\beta$ de l'extrémité de la plaque possédant le trou taraudé étant inférieure à l'inclinaison $\alpha$ de l'autre extrémité de la plaque, cette dernière inclinaison $\alpha$ correspondant sensiblement à l'angle de correction, lequel est généralement de 130°.

Les plaques selon l'invention sont de plus caractérisées par le fait que leur face interne, c'est-à-dire la face en contact avec l'os, présente une concavité qui s'étend jusqu'au pied de l'élément de fixation.

Selon l'art antérieur, l'élément de fixation de telles plaques présentent un forage central, suivi d'un trou taraudé, lui-même suivi d'un chambrage, dont le diamètre correspond à celui de la clef de mise en place de la plaque, le chambrage comportant trois logements prévus pour recevoir les ergots que possède la clef. Ce type de fixation de la clef dans la plaque pour sa mise en place ou son extraction présente des inconvénients ; en particulier les ergots de taille relativement petite se cassent facilement sous l'effet des forces appliquées pour la mise en place de la plaque.

Les plaques selon l'invention sont dépourvues de chambrage ; elles présentent dans l'élément de fixation uniquement un forage, suivi d'un trou taraudé et possèdent sur leur face externe au moins une rainure qui s'étend radialement par rapport à l'axe dudit trou taraudé.

L'invention concerne également une clef pour la mise en place des clou-plaques selon l'invention ; cette clef étant essentiellement constituée :

1) d'une tige munie à une extrémité d'une tête et à l'autre extrémité d'une partie filetée et ;

2) d'un manchon dont l'extrémité opposée à la tête de la tige à une face oblique munie d'au moins un ergot qui coopère avec la rainure de la plaque lors de la mise en place ou de l'extraction de celle-ci, ledit ergot s'étendant radialement par rapport à l'axe du manchon.

L'invention va être maintenant décrite en détail en référence aux dessins annexés sur lesquels :

FIG. 1 est une coupe en élévation d'un clou-plaque selon l'invention ;

FIG. 2 est une vue de droite du clou-plaque selon la figure 1 ;

FIG. 3 est une coupe selon II-II du clou-plaque selon la figure 2 ;

FIG. 4 est une coupe en élévation avec arrachement partiel d'une clef selon l'invention ;

FIG. 5 est une vue de gauche de la clef selon la figure 4 ;

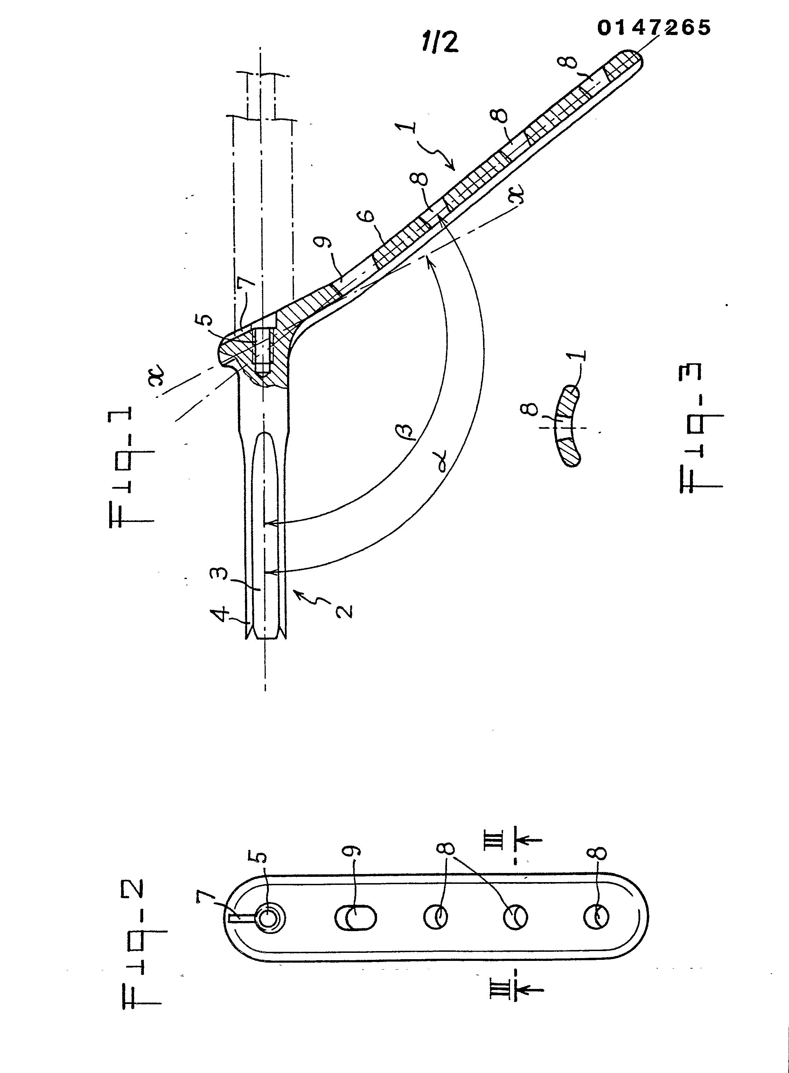

Les figures 1 à 3 représentent un mode de réalisation de la plaque selon l'invention, qui comprend une plaque 1, solidaire d'un élément de fixation 2 ou corps du clou-plaque ; ce corps est cylindrique dans sa partie voisine de la plaque 1 et comporte sur le reste de sa longueur quatre gorges profondes 3 définissant quatre ailettes 4 avantageusement de même longueur ; l'extrémité de chaque ailette est taillée en pointe. Le corps 2 comporte un forage central (non représenté) de diamètre égal à celui-ci de la broche utilisée pour le forage de l'os. Ce forage central est suivi d'un trou taraudé 5. La plaque 1

présente, sur sa face externe 6 au niveau du trou taraudé 5, au moins une rainure 7 qui s'étend radialement par rapport à l'axe du trou taraudé 5. Avantageusement, la plaque selon l'invention présente une seule rainure 7 qui s'étend de la périphérie du trou taraudé 5 jusqu'à l'extrémité de la plaque selon une direction parallèle à l'axe x-x de la plaque.

La plaque est caractérisée par les inclinaisons $\alpha$ et $\beta$, par rapport à l'axe du corps 2, l'inclinaison $\beta$ étant inférieure à l'inclinaison $\alpha$ , cette dernière correspondant à l'angle $\alpha$ de correction. De plus, la plaque comporte des trous 8, dont le nombre varie suivant la longueur de la plaque ; ces trous sont percés selon une direction normale à la plaque. La plaque comporte également un trou 9 percé suivant une direction parallèle à l'axe du corps 2 ou suivant une direction quelconque par rapport à cet axe.

La figure 1 est un mode de réalisation de l'invention et ne limite aucunement la portée de celle-ci qui englobe également les plaques à vis.

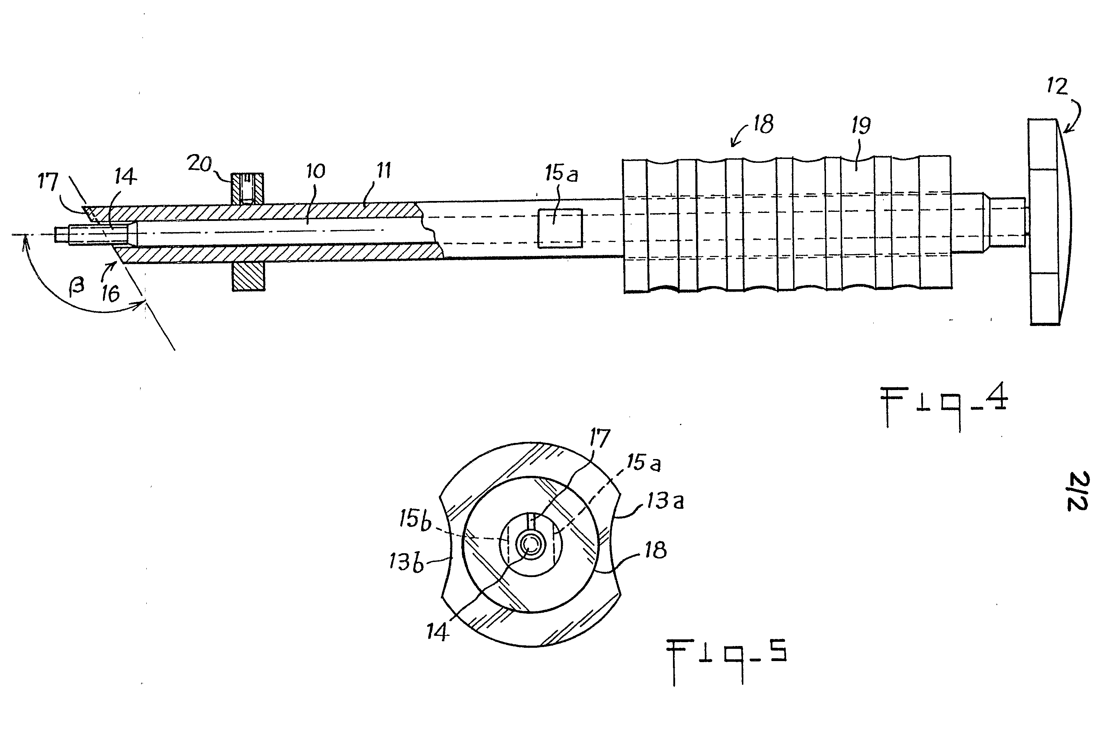

Les figures 4 et 5 représentent la clef de mise en place de la plaque selon l'invention.

Cette clef comprend un porte-clou constitué d'une tige creuse 10 et un manchon 11. La tige 10 est munie à une extrémité d'une tête 12 constituée d'un disque comportant deux parties incurvées 13a et 13b. La tige 10 comprend à son autre extrémité une partie filetée 14 de diamètre et de pas correspondant au filetage du trou taraudé 5 de la plaque représentée sur la figure 1. Le manchon comporte deux méplats 15a et 15b diamétralement opposés ; son extrémité 16, opposée à la tête 12 a une face oblique munie d'au moins un ergot 17 qui s'étend radialement par rapport à l'axe du manchon. La face oblique 16 du manchon fait avec l'axe de celui-ci un angle $\beta$ , identique à l'inclinaison $\beta$ de la plaque. La clef selon l'invention peut également comporter une masse coulissante 18 constituée d'un cylindre creux qui coulisse le long du manchon 11 et possède des nervures 19 appropriées pour une bonne préhension et manipulation. Le manchon 11 comporte éventuellement une collerette 20 qui sert de butée pour la masse coulissante 18.

Pour la mise en place d'un clou-plaque selon l'invention, on pratique d'abord, selon le mode opératoire classique, un forage dans le col du fémur fracturé et ensuite on introduit le clou-plaque de la manière suivante :

On visse l'extrémité 14 du porte-clou dans le trou taraudé 5 de la plaque de manière à faire pénétrer l'ergot 17 dans la rainure 7. On engage ensuite la broche de forage dans le forage central du clou-plaque, puis dans la tige creuse 10 et on introduit le clou-plaque dans le forage en frappant avec un marteau sur la tête 12 de la tige 10 ou en utilisant la masse coulissante 18 lorsque le manchon 11 comporte une collerette de butée 20. Sur la figure 1 on a représenté en pointillés la clef en position d'utilisation.

Grâce à la face oblique 16 et à l'ergot 17 du manchon 11, qui coopèrent avec la face de la plaque ayant l'inclinaison $\beta$ et la rainure 7 de celle-ci, il n'y a pas de risques de rotation du clou-plaque lors de son introduction dans le forage.

D'autre part, les dimensions de l'ergot 17 assurent une bonne robustesse contrairement aux ergots de l'art antérieur.

Pour l'extraction du clou-plaque, on procède de la même façon avec une clef munie d'une masse coulissante 18 et on retire le clou en tapant avec la masse coulissante sur la tête du porte-clou.

0147265

R E V E N D I C A T I O N S
------------------------------

1.    Plaque d'ostéosynthèse comprenant une plaque (1) et un élément de fixation (2), ladite plaque présentant des trous (8) et (9) et un trou taraudé (5), caractérisé en ce qu'elle présente, par rapport à l'axe de l'élément de fixation (2) deux inclinaisons $\alpha$ et $\beta$, l'inclinaison $\beta$ de l'extrémité de la plaque possédant le trou taraudé (5) étant inférieure à l'inclinaison $\alpha$ de l'autre extrémité de la plaque, ladite inclinaison $\alpha$ correspondant sensiblement à l'angle $\alpha$ de correction.

2.    Plaque selon la revendication 1, caractérisée en ce que la face interne de la plaque présente une concavité qui s'étend jusqu'au pied de l'élément de fixation (2).

3.    Plaque selon l'une des revendications 1 ou 2, caractérisée en ce que la face externe de ladite plaque comprend au moins une rainure (7) qui s'étend radialement par rapport à l'axe du trou taraudé (5).

4.    Plaque selon l'une quelconque des revendications 1 à 3, caractérisée en ce qu'elle est du type clou-plaque et en ce que l'élément de fixation (2) est cylindrique dans sa partie voisine de la plaque, comporte sur le reste de sa longueur quatre gorges (3) définissant des ailettes (4) en ce qu'il possède un forage central suivi du trou taraudé (5).

5.    Clef pour ostéosynthèse, caractérisée en ce qu'elle comprend

   1) un porte-clou constitué d'une tige creuse (10) munie à l'une de ses extrémités d'une tête (12) et à l'autre d'une partie filetée (14) ;

   2) un manchon (11) présentant à son extrémité opposée à la tête (12) une face oblique (16) munie d'au moins un ergot (17) qui s'étend radialement par rapport à l'axe du manchon ;

   3) éventuellement une masse coulissante (18).

6.    Clef selon la revendication 5, caractérisée en ce que le manchon comporte en outre une collerette de butée (20).

Fig-2

Fig-1

Fig-3

1/2

0147265

Fig-4

Fig-5

0147265

**0147265**

Office européen
des brevets

**RAPPORT DE RECHERCHE EUROPEENNE**

Numéro de la demande

EP 84 40 2275

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int. Cl.4) |
|---|---|---|---|
| X | FR-A-2 066 417 (A. MENSCHIK) <br> * page 2, lignes 28-33; figures * | 1,2,4 | A 61 B 17/56 |
| Y | | 3 | |
| Y | EP-A-0 091 499 (METALLWERK PLANSEE) <br> * figure 3 * | 3 | |
| A | | 5 | |
| X | FR-A-2 299 006 (R. TORNIER) <br> * page 2, lignes 2-29; figures * | 1,4 | |
| A | | 5 | DOMAINES TECHNIQUES RECHERCHES (Int. Cl.4) |
| Y,D | FR-A-1 443 965 (L. DESCAMPS) <br> * page 2, colonne 2, lignes 4-7; page 4, colonne 2, ligne 51 - page 5, colonne 1, ligne 47; fig- ures 2,11,34,43-49 * | 5,6 | A 61 B |
| A | | 1-4 | |
| Y | US-A-3 439 671 (G. KÜNTSCHER et al.) <br> * en entier * | 5,6 | |

---  -/-

Le présent rapport de recherche a été établi pour toutes les revendications

| Lieu de la recherche <br> LA HAYE | Date d'achèvement de la recherche <br> 15-01-1985 | Examinateur <br> WOLF C.H.S. |
|---|---|---|

CATEGORIE DES DOCUMENTS CITES

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

 

& : membre de la même famille, document correspondant

OEB Form 1503 03 62

Office européen
des brevets

**RAPPORT DE RECHERCHE EUROPEENNE**

Numéro de la demande

EP 84 40 2275

Page 2

| Catégorie | Citation du document avec indication, en cas de besoin, des parties pertinentes | Revendication concernée | CLASSEMENT DE LA DEMANDE (Int. Cl.4) |
|---|---|---|---|
| A | FR-A-2 343 710 (H. BAUDOT et al.)<br>* en entier * | 1,2,4 | |
| A | DE-B-1 046 827 (E. POHL)<br>* colonne 1, lignes 18-29; figures * | 3 | |
| A | DE-C- 935 859 (A.N. WITT)<br>* page 2, lignes 38-41; figures * | 3 | |
| A | US-A-2 937 642 (K.O. LANGE et al.)<br>* colonne 3, lignes 50-66; figures 2,4,5 * | 5 | |
| A | WO-A-8 000 534 (W.-D. OTTE et al.)<br>* abrégé * | 5,6 | **DOMAINES TECHNIQUES RECHERCHES (Int. Cl.4)** |
| A | US-A-4 341 206 (T.R. PERRETT et al.)<br>* abrégé * | 5,6 | |
| A | FR-A-1 354 525 (J. COLMENARES)<br>* en entier * | 1 | |

Le présent rapport de recherche a été établi pour toutes les revendications

| Lieu de la recherche | Date d'achèvement de la recherche | Examinateur |
|---|---|---|
| LA HAYE | 15-01-1985 | WOLF C.H.S. |